# EUROPEAN PATENT SPECIFICATION

(11) **EP 1 067 879 B1**
(45) Date of publication and mention of the grant of the patent: **28.02.2007**
(21) Application number: 99916264.7
(22) Date of filing: 01.04.1999
(51) Int. Cl.: A61B 19/00, A61K 38/48

(54) **METHOD FOR INHIBITING THROMBOSIS IN A PATIENT WHOSE BLOOD IS SUBJECTED TO EXTRACORPOREAL CIRCULATION**
VERFAHREN ZUR THROMBOSEHEMMUNG BEI PATIENTEN DEREN BLUT EXTRAKORPORALER ZIRKULATION AUSGESETZT IST
METHODE D'INHIBITION DE LA THROMBOSE CHEZ UN PATIENT DONT LE SANG EST SOUMIS A UNE CIRCULATION EXTRACORPORELLE

(30) Priority: 01.04.1998 US 53872
(43) Date of publication of application: 17.01.2001
(73) Proprietor: The Trustees of Columbia University in the City of New York, New York, New York 10027 (US)
(72) Inventor: ROSE, Eric, Tenafly, NJ 07670 (US); STERN, David, Great Neck, NY 11020 (US); SCHMIDT, Ann, Marie, Franklin Lakes, NJ 07417 (US); SPANIER, Talia, New York, NY 10025 (US)
(74) Representative: Lawrence, John
(86) International application number: PCT/US1999/007173
(87) International publication number: WO 1999/049803

(56) References cited:
- WO-A-95/34319
- WO-A-96/00577
- WO-A-98/13058
- US-A- 5 443 960
- US-A- 5 472 850
- US-A- 5 839 443
- SPANIER T B ET AL: "Selective anticoagulation with active site blocked factor IXa in synthetic patch vascular repair results in decreased blood loss and operative time." ASAIO JOURNAL, (1997 SEP-OCT) 43 (5) M526-30. , XP000728783
- SPANIER, TALIA B. ET AL: "Active site-blocked factor IXa (IXai): a novel selective anticoagulant for use in cardiopulmonary bypass" SURG. FORUM (1997), 48, 259-261 , XP008003530
- SPANIER, TALIA BARZEL (1) ET AL: "Active-site blocked factor IXa is a selective alternative anticoagulant to heparin in cardiopulmonary bypass." CIRCULATION, (10/21/97, 1997) VOL. 96, NO. 8 SUPPL., PP. I300. MEETING INFO.: 70TH SCIENTIFIC SESSIONS OF THE AMERICAN HEART ASSOCIATION ORLANDO, FLORIDA, USA NOVEMBER 9-12, 1997 , XP008003533
- SPANIER, TALIA B. ET AL: "A novel anticoagulant strategy in peripheral vascular surgery that decreases operative blood loss" SURGICAL FORUM (1998), 49, 333-335 , XP008003541
- SPANIER T B ET AL: "Heparinless cardiopulmonary bypass with active-site blocked factor IXa: a preliminary study on the dog." JOURNAL OF THORACIC AND CARDIOVASCULAR SURGERY, (1998 MAY) 115 (5) 1179-88. , XP008003529
- SPANIER T B ET AL: "Selective anticoagulation with active site-blocked factor IXA suggests separate roles for intrinsic and extrinsic coagulation pathways in cardiopulmonary bypass." JOURNAL OF THORACIC AND CARDIOVASCULAR SURGERY, (1998 NOV) 116 (5) 860-9. , XP008003542
- BENEDICT C R ET AL: "Active site-blocked factor IXa prevents intravascular thrombus formation in the coronary vasculature without inhibiting extravascular coagulation in a canine thrombosis model." JOURNAL OF CLINICAL INVESTIGATION, (1991 NOV) 88 (5) 1760-5. , XP008003522

## Description

### Background of the Invention

Throughout this application, various publications are referenced by author and date. Full citations for these publications may be found listed alphabetically at the end of the specification immediately preceding the claims. The disclosures of these publications fully describe the state of the art as known to those skilled therein as of the date of the invention described and claimed herein.

In cardiopulmonary bypass surgery, one of the critical requirements is the maintenance of blood fluidity and the absence of thrombosis. The cardiopulmonary bypass circuit presents a unique combination of factors favoring the development of a prothrombotic environment. The contact of blood with numerous devices which are associated with this procedure, such as membrane oxygenators and filters, has been implicated in the activation of the intrinsic (contact) pathway of coagulation. Since the bypass circuitry generates a highly-thrombogenic environment, high levels of anticoagulation therapies are required (Edmunds, 1995; Edmunds, 1993; Gravlee et al., 1990; Walenga et al., 1991; DeAnda et al., 1994; Brister et al., 1994; and Chomiak et al., 1993). Traditional intervention to prevent thrombosis in this setting has been the use of heparin. However, the use of heparin causes unacceptable side effects in certain patients. Such side effects may include the development of bleeding, heparin resistance or arterial/venous thrombosis, Despite investigations which have attempted to provide alternatives to the use of heparin, such as thrombin inhibitors (such as hirudin) and dermatan sulphate, no agent has yet been identified to replace or modify its use in clinical cardiac surgery (Walenga et al., 1991; DeAnda et al., 1994; Brister et al., 1994; and Chomiak et al., 1993).

In addition, Benedict et al. (1994) Texas Heart Institute Journal Vol 21, No. 1, pp 85-90, disclose that infusion of Factor IXai at concentrations sufficient to inhibit intravenous coagulation did not produce bleeding significantly different from that in control animals.

Spanier T B et al (ASAIO Journal 1997, 43(5) M526-30) describe selective anticoagulation with active site blocked Factor IXa in synthetic patch vascular repair.

Spanier T B et al (Surgical Forum 1997, 48, 259-261) describe active site-blocked factor IXa (IXai) as a novel selective anticoagulant for use in cardiopulmonary bypass.

T.B. Spanier (Circulation (10/21/97, 1997) Vol. 96, No 8, Suppl. pp 1300: 70^{th} Scientific Sessions of the American Heart Association) describes active site-blocked factor IXa as a selective alternative anticoagulant to heparin in cardiopulmonary bypass.

WO95/34319 describes a method for inhibiting the growth and/or causing regression of tumours by administering a therapeutically effective dose of a procoagulant and a cytokine, preferably TNF-β, TNF-α and/or IL-1.

WO96/00577 discloses analogs of blood factors which are transiently inactive are useful in treatment of diseases characterised by thrombosis.

Spanier T B et al (Surgical Forum 1998, 49, 333-335) describe a novel anticoagulant strategy in peripheral vascular surgery that decreases operative blood loss.

Spanier T B et al (Journal of Thoracic and Cardiovascular Surgery, 1998 May, 115(5) 1179-88) describe heparinless cardiopulmonary bypass with active-site blocked factor IXa.

Spanier T B et al (Journal of Thoracic and Cardiovascular Surgery, 1998 Nov, 116(5) 860-9) describe that selective anticoagulation with active-site blocked factor IXa suggests separate roles for intrinsic and extrinsic coagulation pathways in cardiopulmonary bypass.

WO98/13058 describes a method for treating an ischemic disorder in a subject in which a subject is administered a pharmaceutically acceptable form of a selection antagonist to prevent white blood cell accumulation so as to treat the ischemic disorder in the subject.

Benedict C R et al (Journal of Clinical Investigation 1991 Nov, 88(5) 1760-5) describes an active site-blocked factor IXa which prevents intravascular thrombus formation in the coronary vasculature, without inhibiting extravascular coagulation in a canine thrombosis model.

US 5 839 443 A describes an assay to monitor antithrombic activity of a factor IXa compound infused into the circulation of a patient.

US 5 443 960 A describes a method of detecting APC resistance relevant to the diagnosis of blood coagulation disorders.

US 5 472 850 A describes a quantitative clotting assay for activated factor VII using truncated tissue factor (tTF), a soluble mutant form of (tTF) that retains the cofactor function of TF toward factor VIIa.

### Summary of the Invention

This invention provides the use of a Factor IXmi or a factor IXami for the manufacture of a medicament for inhibiting thrombosis in a patient whose blood is subjected to extracorporeal circulation. Preferably the Factor IXmi or Factor IXami is to be administered at a concentration of from about 0.01 µg/ml plasma to about 250 µg/ml plasma or from about 0.05 µg/ml plasma to about 25 µg/ml plasma. The medicament may be for use when the patient is subjected to extracorporeal blood circulation during transplant surgery or cardiopulmonary bypass surgery.

### Brief Description of the Figures

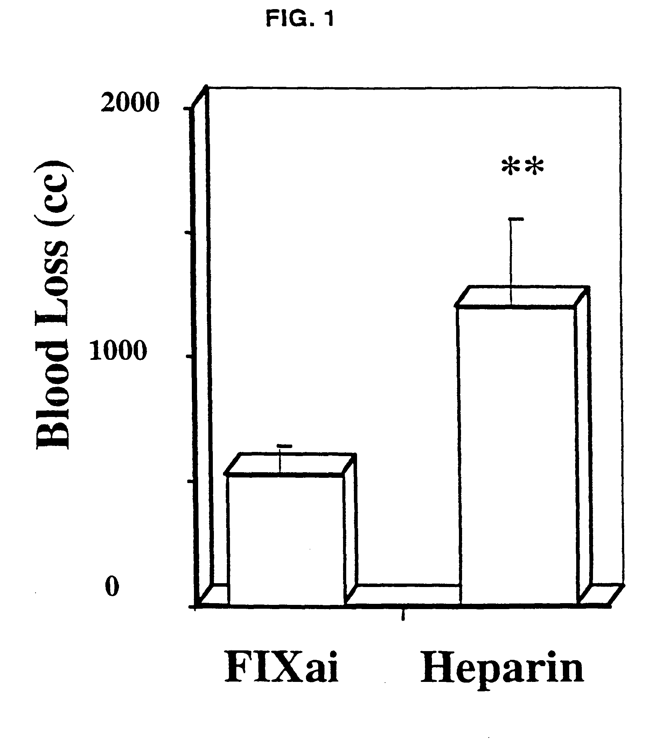

### Figure 1. Blood Loss: Factor IXai vs Heparin

Blood loss in the thoracic cavity was assessed in dogs treated with Factor IXai and dogs treated with heparin. After 1 hour of bypass followed by up to three hours of postoperative observation, blood loss was significantly less in the group receiving Factor IXai compared with the group receiving heparin (**, p<0.05).

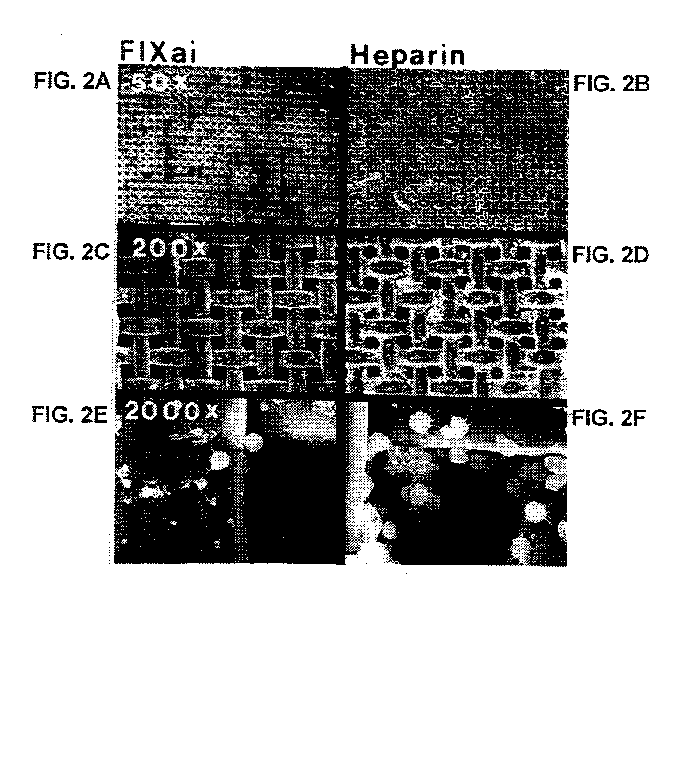

### Figures 2A, 2B, 2C, 2D, 2E and 2F. Scanning Electron Microscopic Analysis of Arterial Filter in Factor IXai vs Heparin

Scanning electron microscopic analysis of the arterial filters is depicted. The Figures 2A, 2C and 2E show serial magnification of the arterial filters in Factor IXai treated dogs (50 x (top), 200x (middle), 2000x (bottom)). The Figures 2B, 2D and 2F show similar views of the heparin treated dogs. As demonstrated in these pictures, cardiopulmonary bypass performed with Factor IXai was associated with similar amounts of clinically inapparent fibrin deposition compared with the use of traditional heparin.

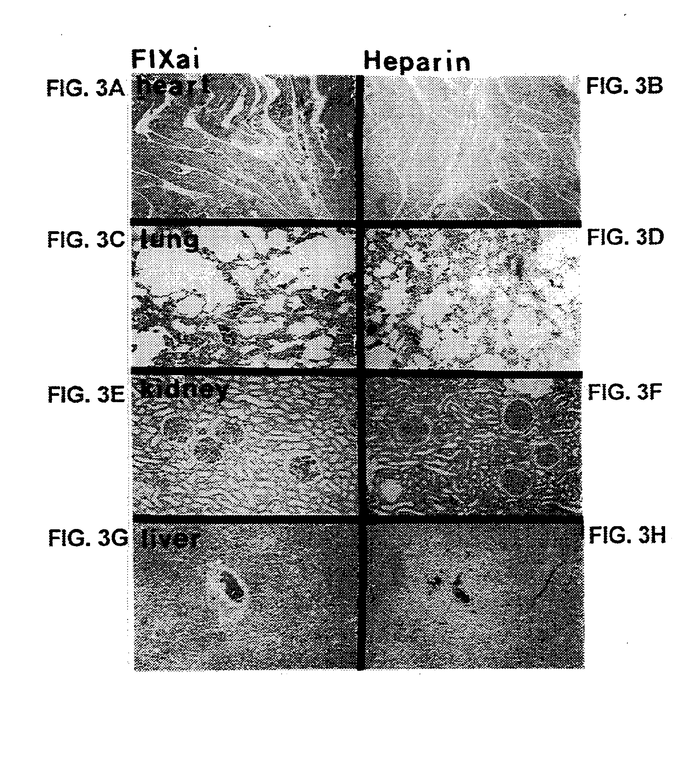

### Figures 3A, 3B, 3C, 3D, 3E, 3F 3G, and 3H. Histologic Examination of End Organs

Hematoxylin/eosin staining of heart, lung, kidney, and liver in dogs treated with Factor IXai are shown in Figures 3A, 3C, 3E, 3G. Figures 3B, 3D, 3F and 3H show samples evaluated in dogs treated with heparin. These studies reveal the absence of fibrin deposition and micro emboli in both groups.

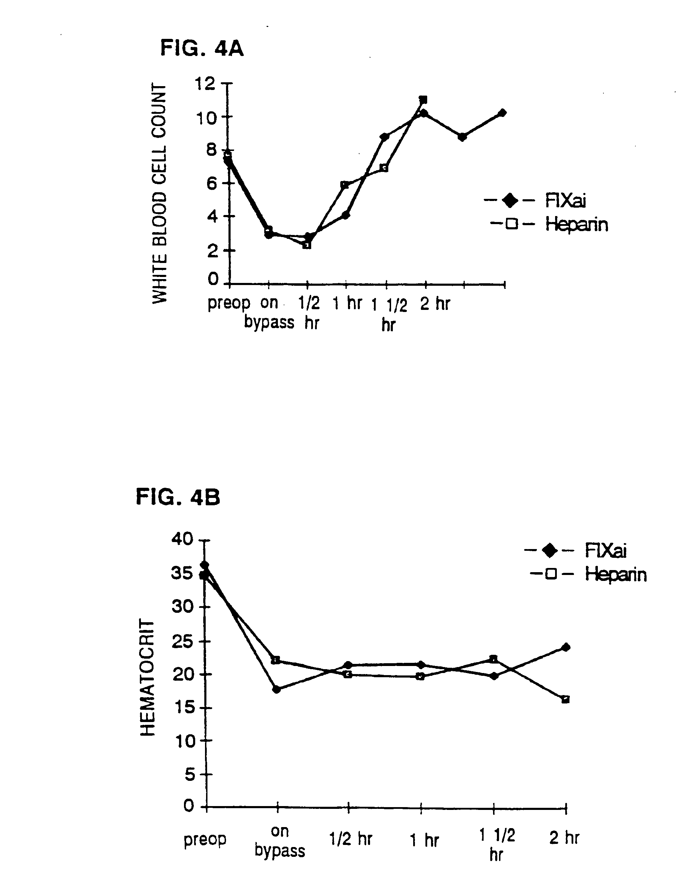

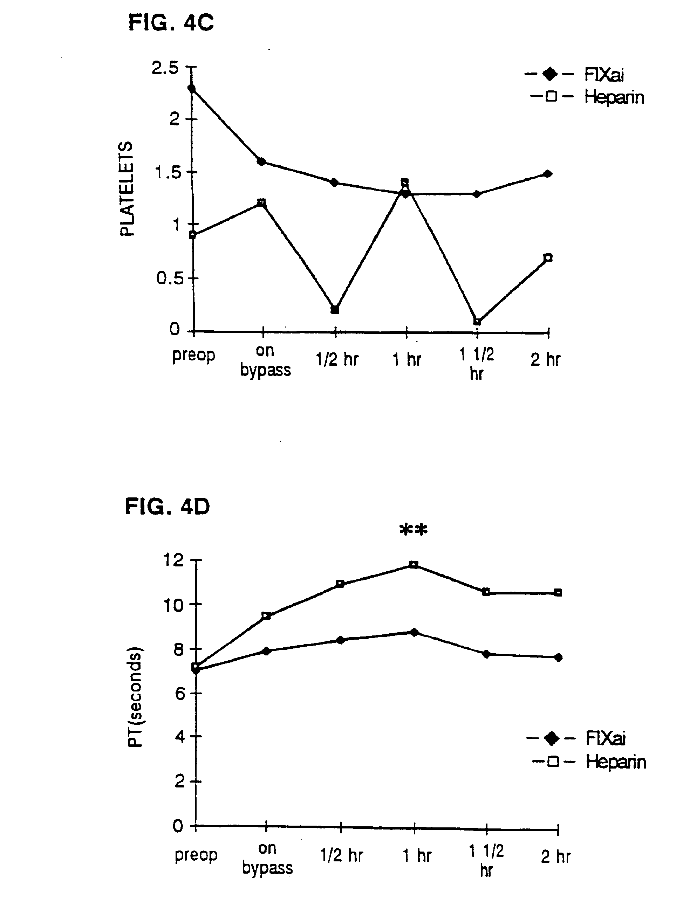

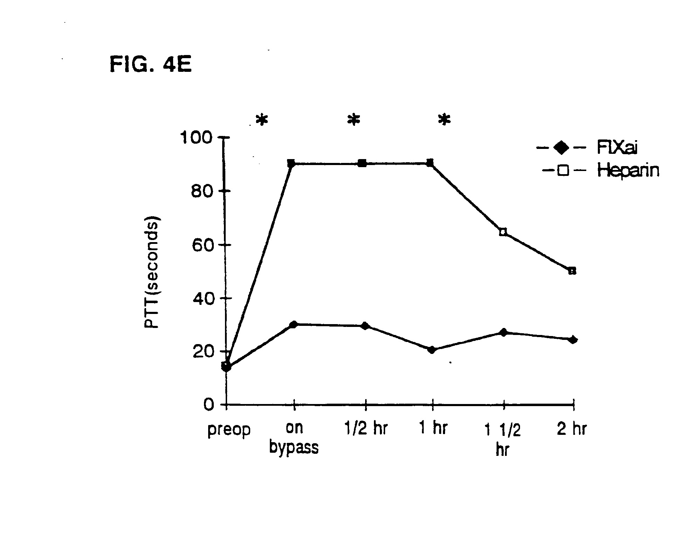

### Figures 4A, 4B, 4C, 4D and 4E. Analysis of Blood Samples in Dogs Treated with Factor IXai and Heparin

Blood samples were drawn at intervals throughout the surgical procedure and evaluated. Similar dilutional decreases in white blood cells, hematocrit, and platelet count were observed in each group. Levels of prothrombin time and partial thromboplastin time remained baseline in Factor IXai treated dogs while there was significant elevation in dogs anticoagulated with heparin. (**p<0.05) {closed diamonds represent Factor IXai; open squares represent heparin}.

### Figures 5A and 5B. Factor IX based Clotting Assay.

A clotting assay was developed to rapidly and reproducibly assess the level of anticoagulation during cardiopulmonary bypass. As shown, this is an assay based on Factor IX deficient plasma and an optimized dose of cephalin to determine the functional anticoagulant effect of Factor IXai.

### Figures 6A and 6B. Blood Loss and Hemostasis.

Active Site-blocked Factor IXa was used in peripheral vascular surgery in rabbits and dogs. The blood loss at the aortotomy suture site was substantially less and the time to achieve hemostasis was decreased in animals treated with Factor IXa.

### Figure 7. Clotting Time vs. Concentration of Factor IXai

By performing a series of dilutions of administered Factor IXai and determining the clotting time, it was determined that the limit of detection of Factor IXai in the assay is 0.08 µg/ml.

### Figure 8. Modified Cephalin Clotting Time (MCCT) vs Time

Determination of MCCT after single dose of Factor IXai in canine cardiopulmonary bypass. After a single clinically-effective dose of Factor IXai (460 µg/kg) in canine cardiopulmonary bypass, the MCCT rises to 80 seconds and is maintained at that level through at least 1.5 hrs of cardiopulmonary bypass.

### Detailed Description of the Invention

This invention provides a use as defined in claim 1. The effective amount of a Factor IXmi or Factor IXami may include from about 0.01 µg/ml plasma to about 250 µg/ml plasma or from about 0.05 µg/ml plasma to about 25 µg/ml plasma or preferably from 0.1 µg/ml plasma to about 5 µg/ml plasma.

This invention further provides that the patient may be subjected to extracorporeal blood circulation during transplant surgery, abdominal surgery, vascular surgery or cardiopulmonary bypass surgery or any surgery in which obligate clamping of a blood vessel is required. The patient may be subjected to extracorporeal blood circulation during any kind of cardiac surgery, including bypass grafting, valve replacement, congential repair heart surgery and heart transplantation. The patient may be a human being. The patient may also be subjected to extracorporeal life support. The patient may be a cardiogenic shock patient. The patient may be undergoing hemodialysis, continuous arterio-venus hemofiltration (CAVH), continuous veno-venous hemofiltration (CVVH), extracorporeal circulatory membrane oxygenation (ECMO), brain surgery, vascular surgery, abdominal surgery, orthopaedic surgery, hip replacement surgery, transplant surgery, or any surgery requiring cardio-pulmonary bypass. The subject may be any patient requiring a mechanical circulatory assistance or ventricle assist device (i.e. LVAD). The subject may be a patient requiring new devices as described in Wickelgren, 1996 such as implantable defibrillators. The subject may also be a patient Buffering with symptoms of systemic lupus erythematosus or TTP (thrombotic thrombocytopenic purpura). The subject may also be a patient requiring plasmapharesis.

As used herein "thrombosis" encompasses formation, development or presence of a blood clot or a blood coagulation which is located inside of a patient or inside of an extracorporeal life support system which circulates blood of the patient. Thrombosis also encompasses the presence of a thrombus which includes a blood clot occluding a blood vessel or formed in a heart cavity. Thrombosis also encompasses the activation of a plasmatic coagulation system in a patient which includes the production of cross-linked fibrin degradation product, protein C, free protein S, coagulation factor II, immunoglobulin G or albumin in the patient. "Thrombosis" also encompasses the formation of a white thrombus which may be composed of platelets and fibrin and is relatively poor in erythrocytes, a disseminated fibrin deposit thrombus or a red thrombus which may be composed of red cells and fibrin. Thrombosis may also include a thromboembolism which is the blocking of a blood vessel by a thrombus which may have been dislodged from a vein.

Thrombosis may occur in areas of retarded blood flow in the patient, at a site of injury or at an abnormal vessel wall in conjunction with an initiating platelet plug. Initiation of clot formation in response to tissue injury is carried out by the extrinsic pathway of clotting. Formation of a pure red thrombus in an area of restricted blood flow or in response to an abnormal vessel wall without tissue injury is carried out by the intrinsic pathway. Intrinsic and extrinsic pathways may converge in a final common pathway characterized by the activation of prothrombin to thrombin and the thrombin-catalyzed conversion of fibrinogen to the fibrin clot.

In the intrinsic pathway, Factor XIa cleaves Factor IX between Arg145 and Ala146 and between Arg 180-Val181, releasing a 35 amino acid peptide and producing Factor IXa having a 145 amino acid light chain (amino acids 1-145) and a 235 amino acid heavy chain (amino acids 181-415) joined by a disulfide bond between cysteine residues at positions 132 and 289. Factor IXa is a serine protease which, when complexed with Factor VIIIa on membrane surfaces, converts Factor X to its active form Factor Xa. The enzyme active site of Factor IXa is located on the heavy chain. Three amino acids in the heavy chain are principally responsible for the catalytic activity, His221, Asp269 and Ser365 (H221, D269 and S365, the catalytic triad). If the amino acids of the heavy chain are numbered from 1 to 235, the catalytic triad is His41, Asp89 and Ser185, and the disulfide bond joining the heavy chain to the light chain is at Cys109 on the heavy chain.

As used herein "a Factor IXa compound" means a compound which inhibits or reduces the conversion of Factor X to Factor Xa by naturally occurring Factor IX. As used herein, a Factor IXa compound may be chosen from one of several subsets. One subset is a chemically modified form of naturally occurring Factor IXa which chemical modification results in the inactivation of Factor IXa (e.g., inactivated Factor IXa, active-site blocked Factor IXa or Factor IXai). Another subset of a Factor IXa compound is any recombinant mutated form of Factor IXa (e.g., a mutein form of Factor IXa, a recombinant Factor IXa with a deletion or Factor IXami). In addition, there are other subsets of a Factor IXa compound which include but are not limited to, for example: (1) nucleic acids, (2) anti-Factor IXa antibodies or fragments thereof, (3) saccharides, (4) ribozymes, (5) small organic molecules, or (6) peptidomimetics.

Thus, a Factor IXa compound may encompass the following: a Glu-Gly-Arg chloromethyl ketone-inactivated human factor IXa, an inactive Christmas factor, a Glu-Aly-Arg chloromethyl ketone-inactivated factor IXa, a glutamylglycyl-arginyl-Factor IXa, a dansyl Glu-Gly-Arg chloromethyl ketone-inactivated bovine factor IXa (IXai), a Factor IXai, a competitive inhibitor of Factor IXa, a peptide mimetic of Factor IXa, a carboxylated Christmas factor, a competitive inhibitor of the formation of a Factor IXa/VIIIa/X complex, a des-γ-carboxyl Factor IX, Factor IX lacking a calcium-dependent membrane binding function, inactive Factor IX including only amino acids 1-47, apoFactor IX including amino acids 1-47, Factor IX Bm Kiryu, a Val-313-to-Asp substitution in the catalytic domain of Factor IX, a Gly-311-to-Glu substitution in the catalytic domain of Factor IX, a Gly-311 to Arg-318 deletion mutant of Factor IX, an anti-Factor IXa antibody, an anti-Factor IXa monoclonal or polyclonal antibody. The Factor IXa compound may also include inactive species of Factor IX described in the references provided herein, especially Freedman et al., 1995; Furie and Furie, 1995; Miyata et al., 1994 and Wacey et al., 1994.

Thus, a Factor IXa compound may be Factor IXa in which the active site is blocked and may be prepared as described in Experimental Details below. The Factor IXa compound may be a Factor IXa which includes post-translational modifications including glycosylation, β-hydroxylation of aspartic acid, γ- carboxylation of glutamic acid and propeptide cleavage. The Factor IXa compound may be concentrated via heparin affinity chromatography or hydrophobic interaction chromatography. The Factor IXa compound may be a genetically engineered, a recombinant Factor IXa in which amino acids at the active site, especially the serine amino acid at the active site, have been altered to render the recombinant Factor IXa functionally inactive, but still capable of competing with intact, native Factor IXa for cell surface binding.

A Factor IXa compound may be an inactive mutein form of Factor IXa which be useful as selective antithrombotic agent. As used herein, "mutein form" of Factor IXa means a protein which differs from natural factor IXa by the presence of one or more amino acid additions, deletions, or substitutions which reduce or eliminate the ability of the protein to participate in the conversion of Factor X to Factor Xa.

The Factor IXa compound may be a proteolytically inactive, recombinant mutein form of Factor IX, which has substantially the same amino acid sequence as normal or native human Factor IX but in which a different amino acid has been substituted for one or more of His221, Aep269 and Ser365. The proteolytically inactive, recombinant mutein form of Factor IXa, may have substantially the same amino acid sequence as normal or native human factor IXa but in which a different amino acid has been substituted for one or more of His41, Asp89 or Ser185 in the heavy chain of Factor IXa. The term "proteolytically inactive" means that the muteins are incapable of converting Factor X to Factor Xa.

This invention uses recombinant muteins of Factor IX which are referred to collectively as Factor Ixmi (i.e., Factor IX mutationally inactivated), or recombinant muteins of Factor IXa which are referred to collectively as Factor IXami. Examples of Factor IXa compounds which are recombinant muteins are as follows:
Factor IXmi (Ser365-Xxx)
Factor IXmi (Asp269-Yyy)
Factor IXmi (His221-Zzz)
Factor IXmi (Ser365-Xxx, Asp269-Yyy)
Factor IXmi (Ser 365-Xxx, His221-Zzz)
Factor IXmi (Asp269-Yyy, His-Zzz)
Factor IXmi (Ser365-Xxx, Asp269-Yyy, His-Zzz)

Factor IXami (Ser365-Xxx)
Factor IXami (Asp269-Yyy)
Factor IXami (His221-Zzz)
Factor IXami (Ser365-Xxx, Asp269-Yyy)
Factor IXami (Ser365-Xxx, His221-Zzz)
Factor IXami (Asp269-Yyy, His-Zzz)
Factor IXami (Ser365-Xxx, Asp269-Yyy, His-Zzz)
wherein Xxx is any one of the standard amino acids other than serine, Yyy is any one of the standard amino acids other than aspartic acid, and Zzz is any of the standard amino acids other than histidine. Preferred recombinant muteins are Factor IXmi (Ser365-Ala) and Factor IXami (Ser365-Ala).

Factor IXmi and Factor IXami are functionally similar to Factor IXai in terms of their ability to establish effective anti-coagulation intravascularly and in ex vivo equipment connected to the blood stream while permitting retention of effective hemostasis. The advantages of Factor IXmi and Factor IXami over Factor IXai are the following:
- Factor IXmi and Factor IXami can be produced directly in a genetically engineered organism, thus avoiding several processing and purification steps with their attendant losses, thereby improving product yield.
- The cost of production of Factor IXmi and Factor IXami in an appropriate genetically engineered organism is lower than the cost of production of Factor IXai from human plasma.
- Factor IXmi and Factor IXami, produced in a genetically engineered organism, will not be subject to the risk of contamination with various infectious agents such as viruses or prions (for example, the agents for HIV disease and for bovine and/or human spongiform encephalopathies).
- Factor IXmi and Factor IXami, being less different from wild-type human Factor IX and Factor IXa than chemically modified Factor IXai, will have a lower probability of eliciting an immune response in patients who are dosed with the modified protein for extended periods of time, thereby reducing the risk of delayed type hypersensitivity reactions and improving patient safety for indications such as anticoagulation in hemodialysis that will require repeated, long-term use.

The recombinant muteins of this invention can be produced by known genetic engineering techniques, using as the starting material recombinant cDNA encoding Factor IX in an appropriate cloning vector. For example, a starting material which may be used in the production of a Factor IXa compound is the product of Example 5 of U.S. Patent No. 4,770,999, i.e., a recombinant plaque of E. coli infected with a bacteriophage M12mp11 Pst vector containing cDNA corresponding to the entire sequence of recombinant Factor IX ligated to Pst adapters. The recombinant plaques are used to prepare single-stranded DNA by either the small-scale or large-scale method described in Sambrook et al., Molecular Cloning. A Laboratory Manual, Second Edition, Cold Spring Harbor Press, 1989, pages 4.29-4.30 and 4.32.

The single-stranded M13mp11 containing Factor IX cDNA is then used to carry out oligonucleotide-mediated mutagenesis using the double primer method of Zoller and Smith as described in Sambrook et al., 1989, pages 15.51-15.73. Mutagenic primers which can be used include the following:
1) Oligonucleotides for producing Factor IXmi(Ser365-Xxx)
   3'-W ACA GTT CCT CTA XXX CCC CCT GGG GTA V-5'
   where
   W is T, 3'-GT or 3'-AGT
   V is C, 3'-CA, or 3'-CAA
   XXX is the complement to a DNA codon for any one of the standard amino acids other than serine.
2) Oligonucleotides for producing FACTOR IXmi (Asp269-Yyy)
   3'-W TTC ATG TTA GTA YYY TAA CGC GAA GAC V-5'
   where
   W IS A, 3'=TA, OR 3'-TTA
   V is C, 3'-CT, or 3'-CTT
   YYY is the complement to a DNA codon for any one of the standard amino acids other than aspartic acid and cysteine.
3) Oligonucleotides for producing Factor IXmi (His221-Zzz)
   3'-TTA CAT TGA CGA CGG ZZZ ACA CAA CTT TGA CCA-5'
   where
   W is A, 3'-AA, or 3'-TAA
   V is C, 3'-CC, or 3'-CCA
ZZZ is the complement to a DNA codon for any one of the standard amino acids other than histidine and cysteine.

Oligonucleotide primers for producing the preferred Factor IXmi of this invention, Factor IXmi (Ser365→Ala), are those of No. 1 above wherein XXX is the complement of a codon for alanine, i.e., 3'-CGA, 3'-CGC, 3'-CGT or 3'-CGC. A specific primer for producing Factor IXmi (Ser365-Ala) is:
3'-GT ACA GTT CCT CTA CGA CCC CCT GGG GTA C-5'

A skilled artisan would recognize and know how to carry out the remaining steps of oligonucleotide-mediated mutagenesis as follows:
- Hybridization of mutagenic oligonucleotides to the target DNA.
- Extension of the hybridized oligonucleotides to the target DNA.
- Transfection of susceptible bacteria.
- Screening of plaques for the desired mutation.
- Preparation of single-stranded DNA from a mutant plaque.
- Sequencing the single-stranded DNA.
- Recovery of double-stranded Factor IXmi cDNA.
- Inserting the double-stranded Factor IXmi cDNA into the expression vector used by Kaufman (for example).
- Expression of Factor IXmi.
- Treating the Factor IXmi with Factor XIa to produce Factor IXami.

In addition to the compounds disclosed herein having naturally-occurring amino acids with peptide or unnatural linkages, the present invention also provides for other structurally similar compounds such as polypeptide analogs with unnatural amino acids in the compound. Such compounds may be readily synthesized on a peptide synthesizer available from vendors such as Applied Biosystems, Dupont and Millipore.

This invention is illustrated in the Experimental Detail section which follows. These sections are set forth to aid in an understanding of the invention but are not intended to, and should not be construed to, limit in any way the invention as set forth in the claims which follow thereafter.

### EXPERIMENTAL DETAILS

Selective inhibition of the intrinsic pathway of coagulation is one possible way to avoid the use of heparin in extracorporeal circulation. Leaving intact the tissue factor-mediated extrinsic pathway of coagulation (initiated by tissue factor-VIIa) may obviate both adverse bleeding and potential prothrombotic side effects of heparin. These side effects are found in certain sensitized patients such as patients with heparin-induced thrombocytopenia. In these cases, heparin may be associated with a prothrombic situation.

The coagulation Factor IX/IXa is a single chain vitamin K-dependent coagulation protein. Limited proteolysis of Factor IX results in a cleavage product which is a two-chain serine protease, Factor IXa, which requires association with the cell surface and the cofactor VIIIa in order to express and exhibit procoagulant activity (Gurewich et al., 1979; Gitel et al., 1977; and Benedict et al., 1991). Factor IXa has an important role in coagulation. When clotting is triggered in the intravascular space, (i.e., thrombosis), it is initiated in the presence of low amounts of tissue factor. In this case, Factor IX becomes preferentially activated to Factor IXa, which then feeds into the rest of the procoagulant cascade, leading to fibrin formation. In contrast, activation of Factor X by tissue factor is less favored by at least an order of magnitude under these conditions. Extravascular coagulation, especially protective hemostasis, occurs in the presence of large amounts of tissue factor on mesenchymal cells and the role of Factor IXa is much less important when direct activation of Factor X occurs. Factor IXa is essentially bypassed. Thus, it may be hypothesized that inhibition of Factor IXa participation in coagulation could provide a selective means of anticoagulation by inhibiting intravascular thrombosis without impairing extravascular hemostasis.

Studies have shown rapid clearance of Factor IX from the intravascular space, the association of infused and endogenous Factor IX with the vessel wall (Thompson, 1986 and Stern et al., 1987) and in vitro studies have demonstrated Factor IX binding to endothelium and platelets. (Heimark and Schwartz, 1983; Stern et al., 1983; and Ahmad et al., 1989) Studies have been performed to characterize the molecular basis of this coagulation protein-cell surface interaction. At the level of the ligand, the amino-terminal gamma-carboxyglutamic acid-containing domain of Factor IX has been shown to be essential for cell surface binding (Toomey et al., 1992; Astermark et al., 1991; Derian et al., 1989; and Ryan et al., 1989). At the level of the cell surface site, previous studies have demonstrated that Factor IX binding involves a protease-sensitive polypeptide. This polypeptide in endothelial cell studies appears to have an Mr≈150 kDa (Rimon et al., 1987) and on platelets appears to have an Mr≈250 kDa(London and Walsh, 1992).

Factor IXa may be capable of binding to cellular interaction sites in the vessel wall. It is possible that such sites may be a target for therapeutic intervention in certain thrombotic disorders such as cardiopulmonary bypass.

Studies have demonstrated that Factor IX/IXa may contribute to thrombosis. It was found that Factor IXa resulted in formation of thrombi (Gurewich et al., 1979) and that Factor IXa has potent thrombogenic properties in the Wessler stasis model of thrombosis (Gitel et al., 1977). Factor IXa participates in procoagulant pathways as a component of the Factor IXa-VIIIa-X activation complex (intrinsic pathway of coagulation). Multiple studies in vitro demonstrated that use of active site-blocked IXa (dansyl-glutamyl-glycyl-arginyl Factor IXa, or IXai) prevented the assembly of IXa into this critical complex (Chomiak et al., 1993; Thompson, 1986; Lollar and Fass, 1984; Stern et al., 1985; and Ahmad et al., 1989). Studies have demonstrated the functionally-active site of Factor IXa (Astermark et al., 1992 and Ahmad et al., 1992). A role for active site-blocked Factor IXa(Factor IXai) has been demonstrated in preventing coronary artery thrombosis in a canine model in which thrombosis is initiated by the introduction of electric current. Extravascular hemostasis was secured, as no untoward bleeding was detectable in an incisional wound model(Benedict et al., 1991). Therefore, Factor IXai may be an ideal antithrombotic agent for use in cardiopulmonary bypass surgery. Specifically, the extrinsic pathway of coagulation would be unaffected and thus the patient would not be predisposed to excess bleeding. Furthermore, other limiting side effects of heparin would be precluded.

In the studies described below, active site-blocked Factor IXa is shown to be a safe and effective antithrombotic agent in a canine model of coronary artery bypass and surgery. An aortotomy was performed in order to stimulate typical cardiac procedures. Pathways regulating control extravascular hemostasis appeared intact because significantly less bleeding was noted in dogs treated with Factor IXai compared with dogs treated with heparin. Furthermore, model studies of cardiopulmonary bypass utilizing active site-blocked Factor IXa further support the efficacy and safety of the present invention.

### MATERIALS AND METHODS

Factor IX/IXa was purified from human plasma according to previously-published methods (Benedict et al., 1991 and Stern et al., 1987) and inactivated using glu-gly-arg-chloromethylketone as described (Benedict et al., 1991 and Lollar and Fass, 1984). Purity of the reagents was then ascertained using SDS-PAGE and standard clotting assays (Benedict et al., 1991 and Lollar and Fass, 1984).

### Animal studies

Cardiopulmonary bypass in dogs (each dog weighing approximately 15 kgs) and baboons (each baboon weighing approximately 11 kgs) (Daly et al., 1988 and Bernabei et al., 1995) was instituted and maintained for one hour with cooling to 32°C. An aortotomy was performed in order to simulate cardiac surgery procedures. Animals were then weaned from bypass and blood loss was monitored for up to 3 hours.

Animals received either heparin (at a standard dose of 300 IU/kg and protamine (2 mg/kg) reversal) or active site-blocked Factor IXa (at different doses as indicated below).

Multiple parameters were assessed in all animals (receiving either heparin or Factor IXai) as follows:
1. In order to test for evidence of fibrin deposition in the bypass circuitry, the cardiopulmonary bypass tubing and filters were removed at the end of bypass and subjected to analysis by scanning electron microscopy in order to detect possible evidence of fibrin deposition.
2. After sacrifice of the dogs or the baboons, necropsy was performed and the heart, lungs, liver and kidneys were removed. These organs were fixed in formalin and examined by Hemotoxylin & Eosin staining and immunofluorescence for evidence of clot formation or fibrin deposition as well as for the presence of microemboli.
3. Routine hematologic analysis was performed prior to initiation of cardiopulmonary bypass and at every 30 minute interval during the cardiopulmonary bypass in order to determine hemoglobin levels, hematocrit levels, levels of platelets and fibrinogen, white blood cell count, prothrombin time, activated partial thromboplastin time and activated clotting time.
4. Continuous hemodynamic measurements were performed prior to, during and for up to 3 hours after the institution of cardiopulmonary bypass.
5. Blood loss was quantitated in the thoracic cavity by collecting all blood in the area during the cardiopulmonary bypass itself and for up to three hours after completion of cardiopulmonary bypass.
6. The extent of activation of coagulation in this model was determined in order to determine the contribution of thrombin generation and fibrinolysis, which occurs in the setting of treatment with heparin, and the contribution of Factor IXai to coagulation. For the canine studies, measurement of thrombin-anti-thrombin complex (or TAT, commercially available from Behring Diagnostics, Boston MA) was utilized as a measure of thrombin generation. This assay was cross-reactive with dog plasma. TAT was measured in animals/group of heparin or Factor IX-ai treated dogs prior to institution of cardiopulmonary bypass/IXai treatment and every 30 minutes during cardiopulmonary bypass and every 60 minutes after cardiopulmonary bypass terminated until the animal was sacrificed at 3 hours. In baboon studies, TAT was measured as described herein and prothrombin fragment 1+2(F ₁₊₂ ; Behring Diagnostics) was measured. This assay cross-reacts with standards obtained from human plasma.
7. Markers of fibrinolysis were assessed to identify the extent to which excess fibrinolysis generated during treatment with heparin likely contributed to increased bleeding. These parameters were directly compared with those obtained in dogs treated with Factor IXai. Levels of d-dimers were assessed at the same time points measured for TAT.

### RESULTS

### Example 1: Canine model of cardiopulmonary bypass

Four dogs were treated with standard doses of heparin (300 IU/kg followed by protamine 2 mg/kg) and five dogs were treated with Factor IXai. In dogs (3 total) treated with 460 µg/kg (single intravenous infusion just prior to the initiation of cardiopulmonary bypass), there was no evidence of excess pressure in the cardiopulmonary bypass circuit or gross clot formation in the tubing. These results were similar to those observed in the dogs treated with heparin. Similarly, systemic hemodynamic profiles were similar in both groups throughout the procedure suggestive of the absence of clinically-relevant thromboses within the bypass circuitry.

Blood loss in the thoracic cavity was significantly less in the group receiving Factor IXai compared with the dogs receiving heparin (Figure 1). After four hours of observation (one hour during cardiopulmonary bypass itself) and three hours following the termination of cardiopulmonary bypass, dogs receiving Factor IXai accumulated 600 ml of blood within the thoracic cavity. This was in marked contrast to dogs receiving standard doses of heparin in which 1000 ml of blood was quantitated in the thoracic cavity (p<0.01).

Consistent with these data and the hypothesis that selective inhibition of the intrinsic pathway of coagulation would leave unaffected the tissue factor-mediated extrinsic pathway of coagulation, dogs that received Factor IXai were observed to have hemostatic clot along the cut surface of the sternum and in surgical tissue planes. However, the dogs treated with heparin did not have these hemostatic markers.

In order to detect pathological quantities of deposited fibrin within the bypass circuits, the tubing and filters were immediately removed after cardiopulmonary bypass and analyzed by scanning electron microscopy. As shown in Figure 2, cardiopulmonary bypass performed with Factor IXai resulted in similar amounts of fibrin deposition (left panel) compared with cardiopulmonary bypass performed with the use of traditional heparin (opposite). The amount of fibrin deposition in both cases was clinically-inapparent.

In addition to microscopic examination of the bypass material, organs removed at necropsy were examined by microscopy with Hematoxylin/eosin staining. These studies revealed the absence of fibrin deposition and microemboli in the liver, lungs, kidneys and myocardium as shown in Figure 3.

Analysis of blood samples revealed similar dilutional decreases in hematocrit, platelet count and fibrinogen levels in both groups of animals treated with either Factor IXai or heparin (Figure 4).

As demonstrated in Figure 4, Prothrombin time (PT) remained at about the level of the control in the Factor IXai-treated group (7.9 + 0.1 sec) and activated Partial thromboplastin time (PTT) was mildly elevated (30.4 ± 11 secs). As expected, heparin-treated dogs had significant elevation of PT and PTT (11.9±0.6 secs and >90 secs, respectively). ACT (Activated clotting time) used in cardiopulmonary bypass to quickly assess the level of anticoagulation with heparin (ideal > 480 secs in human subjects) was >400 secs in the heparin-treated group, but unchanged in the Factor IXai-treated group.

In order to determine the optimal dose of Factor IXai that was necessary to safely prevent thrombosis while securing extravascular hemostasis, different doses of Factor IXai were utilized in the canine model. These data revealed that a dose of 600 µg/kg (one dog) produced no evidence of clotting in the bypass circuitry. However, there was evidence of increased bleeding in this group (900 ml of blood in dogs treated with Factor IXai) compared with dogs that received a lower dose (460 µg/kg) of Factor IXai. A third group of dogs (1100 ml of blood in dogs) treated with heparin also had evidence of increased bleeding. These data suggested that the 600 µg/ml dose of Factor IXai was excessive since the lower dose (at least 460 µg) was successful.

In order to determine the minimal dose of Factor IXai needed to prevent clotting in the bypass circuitry, one dog was treated with Factor IXai at a dose of 300 µg/kg. This experiment demonstrated that there was no evidence of improper clotting of the bypass circuitry. In addition, blood loss in the thoracic cavity was 600 ml of blood compared with 1100 ml of blood in the thoracic cavity of dogs treated with standard doses of heparin.

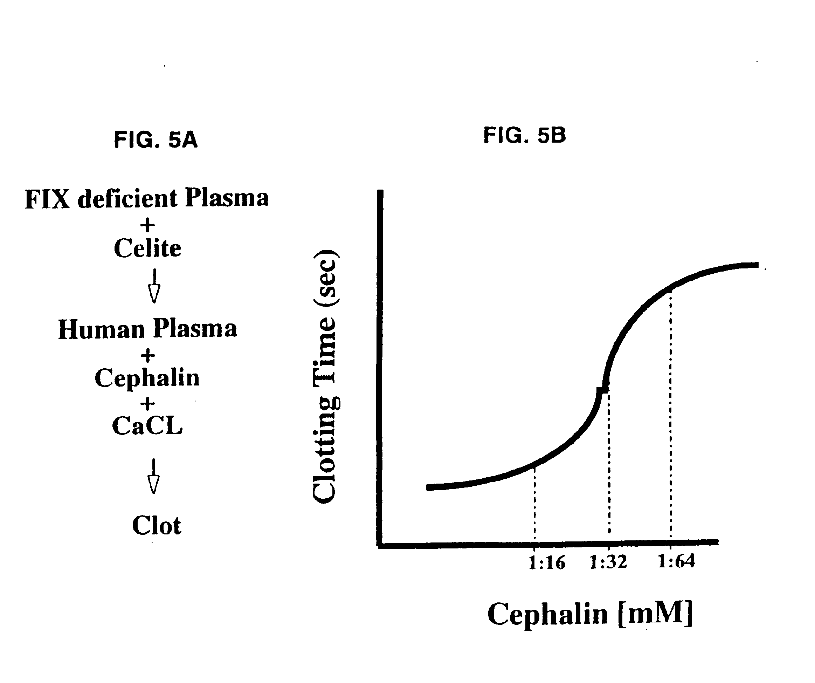

As indicated above, there was an inability to assess the effectiveness of anticoagulation via measurement of activated clotting time in dogs treated with Factor IXa. Thus, a clotting assay was developed in order to determine the functional effectiveness of Factor IXai rapidly and reproducibly, thereby providing a means to assess the level of anticoagulation frequently during cardiopulmonary bypass. It is necessary to be able to frequently determine the ability of blood of a human patient to coagulate throughout the performance of cardiopulmonary bypass surgery or any majory surgery. The sensitivity of the assay was dependent on the use of Factor IX-deficient plasma and an optimized dose of cephalin (source of phospholipid) as shown in Figure 5. Comparison of plasma from dogs treated with Factor IXai compared with control dog plasma revealed a four-fold increase in clotting time. This assay therefore provided a means of rapidly determining the extent of anticoagulation in animals treated with Factor IXai. Further studies may be performed to determine the desired extent of increased clotting time in this assay in order to achieve maximal antithrombotic effects, while maintaining intact the pathways of extravascular hemostasis.

### Example 2: Baboon model of cardiopulmonary bypass

Studies in baboon models of cardiopulmonary bypass are effective and predictive for future testing of active site-blocked Factor IXa in human subjects. Adequate pre-clinical data in primates is expected to more closely predict expected results in humans. Experiments have been performed in baboons. In one experiment, a baboon received Factor IXai (460 µg/kg) and a second baboon received heparin/protamine (300 IU/heparin followed by protamine [2 mg/kg]). The data from these trials revealed that there was no evidence of pathologic clotting in the bypass circuitry observed in either group of animals. Similar to the results from the case of dogs treated with Factor IXai, there was evidence of significantly decreased blood loss in the thoracic cavity of baboons treated with Factor IXa (320 ml of blood) compared with baboons treated with standard doses of heparin (600 ml of blood).

Preliminary hematologic analysis in the baboons shows that there are similar dilutional decreases in hematocrit, white blood cell counts and platelets as seen in the dog trials. Other studies were completed to investigate the effects of Factor IXai in baboons and to characterize the hemostatic findings.

### DISCUSSION

The use of heparin has been associated with multiple side effects in certain patients undergoing cardiopulmonary bypass. The side effects include excessive bleeding (at least in part due to excess fibrinolysis), heparin resistance (potentially requiring use of anti-thrombin III to achieve desire heparin effects), heparin-induced thrombocytopenia (with potential for either excess bleeding or clotting in the arterial and/or venous system) and need for reversal with protamine.

Data from studies of the canine and baboon models of cardiopulmonary bypass suggest that in patients in whom heparin is relatively contraindicated, the use of active site-blocked Factor IXa or (Factor IXai) as an antithrombotic agent may be useful to prevent pathologic clotting in the circuitry. This clotting may be lessened by the inhibition of the intrinsic pathway of coagulation. However, securing the functional responsiveness of the extrinsic (extravascular) pathway of coagulation is critical in minimizing excess blood loss due to cut vessels of the sternum, etc. Furthermore, obviating the use of heparin will be useful to minimize the excessive fibrinolysis generated by its use at doses required to maintain an activated clotting time > 480 secs during cardiopulmonary bypass.

The use of Factor IXai has been shown to be a safe means of inhibition of thrombosis during cardiopulmonary bypass and a range of other cardiac and surgical procedures.

In canine and baboon cardiopulmonary bypass models, the selective inhibition of the intrinsic/contact system of coagulation has been demonstrated. Maintenance of the extrinsic/tissue factor mediated pathway allows for the successful maintenance of patency of the cardiopulmonary bypass circuit while allowing extravascular hemostasis. The possible applications of this therapeutic intervention extend well beyond the cardiopulmonary bypass setting.

For example, extracorporeal life support with extracorporeal membrane oxygenation (ECMO)(Magovern, 1995) is being used more frequently to support adult patients who develop cardiogenic shock. Studies have demonstrated that this intervention provides excellent oxygenation and hemodynamic support in this critically ill population. However, the morbidity and mortality associated with this procedure remains high. Central to the life-threatening complications associated with extracorporeal membrane oxygenation is the unavoidable need for systemic heparinization with its attendant hemorrhagic complications (Muehrcke et al., 1995). Heparin-bonded circuits have provided an alternative to full heparinization with some success, but this model still carries the significant risk of bleeding and thrombosis (Perchinisky et al., 1995 and Atkinson et al., 1992). Selective intravascular anticoagulation with Factor IXai would logically be an ideal alternative to traditional heparinization.

Baboons may be maintained on an extracorporeal membrane oxygenation circuit for up to one week with anticoagulation initiated by Factor IXai, in lieu of heparin. Similarly, active site-blocked Factor IXa may also be useful in any setting requiring the contact of blood with extracorporeal circuitry, such as plasmapheresis, renal hemodialysis, continuous anterio-venus hemofiltration (CAVH), veno-venus hemofiltration (CVVH), extracorporeal circulatory membrane oxygenation (ECMO), brain surgery, vascular surgery, abdominal surgery, transplant surgery, any procedure in which systemic anticoagulation is routinely required, and any procedure in which a patient requires a mechanical circulatory assistance, ventricle assist device, artificial heart, left/right ventricle assist device or a similar biomedical device.

Another potential indication for the use of active site-blocked Factor IXa as an anticoagulant involves the surgical intervention of intracranial aneurysms. Surgery for aneurysm correction must be performed without the use of systematic anticoagulation because the risk of hemorrhagic complications associated with the use of heparin during this procedure are unacceptable. A "watershed" portion of the brain matter immediately adjacent to the aneurysm is therefore sacrificed with the surgical clipping of the aneurysm as tributary vessels thrombose and thus infarct adjacent brain tissue. This procedure may be performed with local infusion of Factor IXai to allow intravascular anticoagulation, thus maintaining blood flow through tributaries of the clipped vessel to the brain matter around the aneurysm. Meanwhile, extravascular/tissue factor mediated hemostasis would ensure that a hemorrhagic infarction did not occur. A murine model of stroke may be studied to determine if local infusion of Factor IXai will limit the "watershed" region around a clipped cerebral vessel while preventing hemorrhage.

### Example 3: Applications of Active site-blocked Factor IXa (IXai) as an antithrombotic agent in cardiopulmonary bypass

One of the potential uses of Factor IXai might be in vascular surgery. At the present time, when vascular surgery is performed, a polytetrafluoroethylene (PTFE) graft is used to reconstitute an injured native blood vessel. At the time of surgery, heparin therapy is indicated in order to prevent thrombosis at the site of the graft. One of the consequences of this intervention, however, is increased bleeding at the needle hole sites (where graft connects with the native vessel). This may increase duration of anesthesia since these bleeding sites must be secured and hemostasis confirmed prior to conclusion of the operation. These interventions, by increasing duration of anesthesia, may actually increase morbidity, especially in predisposed patients.

Studies have been performed with a standing protocol in which a graft (PTFE) is created and then tests of the effects of certain interventions are performed on the graft. One intervention, use of Factor IXai, resulted in no evidence of thrombosis and greatly minimized bleeding about the sites of the needle holes. To follow are results of data:
Studies to test the role of Factor IXai as a novel antithrombotic agent in this setting have been performed in a rabbit model of vascular repair. An infrarenal abdominal aortotomy was performed and reconstructed with a Polytetrafluoroethylene (PTFE)graft (2X6 mm).

### Methods:

A PTFE graft was placed as above and preweighed gauze pad placed near the needle holes. IXai was administered as an intravenous bolus dose at the time of the graft placement. Time to bleeding was observed and weight of gauze after bleeding ceased determined to quantitate the amount of blood loss:

### Results of studies:

| | | |
|---|---|---|
| **Factor IXai:** | **360 microgram/kg:** | |
| | | |
| | **Time to stop bleeding:** | **3 minutes** |
| | | |
| | **Weight of gauze after tare:** | **5.54 grams** |
| | | |
| **Factor IXai:** | **260 microgram/kg:** | |
| | | |
| | **Time to stop bleeding:** | **3 minutes** |
| | | |
| | **Weight of gauze after tare:** | **2.09 grams** |
| | | |
| **Heparin:** | **25 U/kg:** | |
| | | |
| | **Time to stop bleeding:** | **4 minutes** |
| | | |
| | **Weight of gauze after tare:** | **8.61 grams** |

Even at the lowest effective dose of Factor IXai, there was no evidence of thrombus in the PTFE graft. These results suggest that use of Factor IXai may be beneficial in vascular surgery by preventing thrombosis peri-PTFE graft and, yet, at the same time, securing vascular hemostasis.

Regarding interpretation of the results of the PTFE graft placement in rabbits with either Factor IXai or standard heparin therapy:
The effectiveness of Factor IXai in preventing intravascular thrombosis at the site of PTFE graft placement is dose dependent. At the lowest dose studied to date, 260 microgram/kg of IXai, there is NO evidence of thrombosis in the setting of minimal bleeding complication - which is clearly superior to the bleeding observed using the standard dose of heparin. However, as the dose of Factor IXai approaches 360 microgram/kg, there is similar prevention of thrombosis, but increased bleeding. These data suggest that at higher doses of Factor IXai, the anticoagulant effect is more potent than the selective antithrombotic effect at lower doses (of Factor IXai).

### Use of Active Site-blocked Factor Ixa(IXai in peripheral vascular surgery).

### A. Rabbit aortotomy

To date, 37 rabbits have been completed using the New Zealand rabbit model. 21 rabbits have been treated with Factor IXai 300 microgram/kg and 16 rabbits received standard doses of heparin, in this case (50 U/kg). The results of these studies show that the blood loss at the aortotomy suture site was substantially less in the rabbits treated with Factor IXai vs. heparin:

| | |
|---|---|
| Factor IXai | 2.7±2.5 gms vs |
| heparin | 6.9±4.4 gms p<0.05 |

Similarly, the time to achieve hemostasis was decreased in animals treated with Factor IXai:

| | |
|---|---|
| Factor IXai | 120±38 seconds |
| heparin | 176+46 seconds p<0.05 |

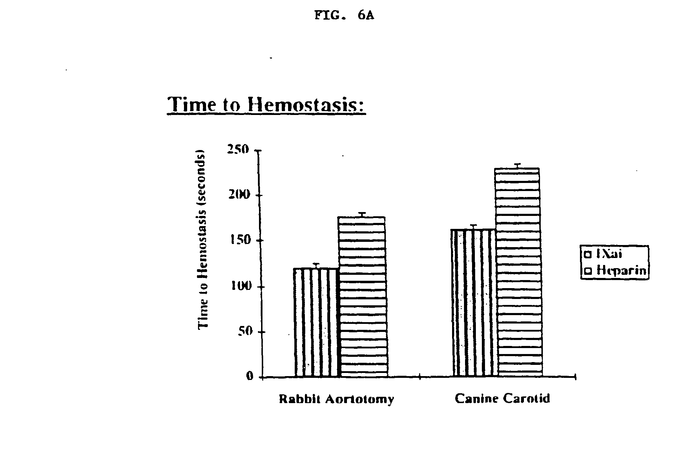

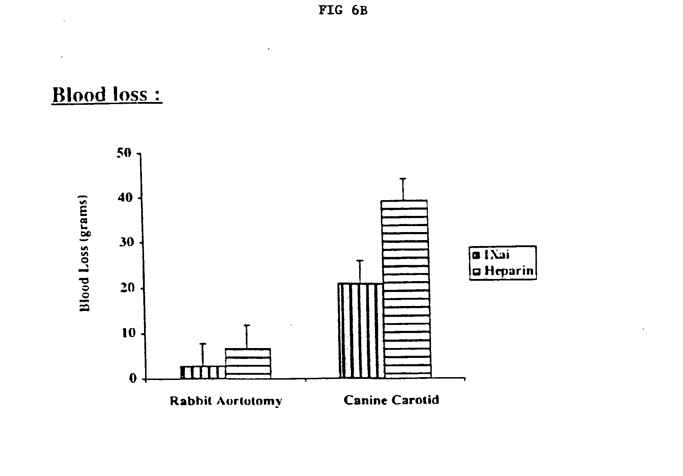

(See Figures 6A and 6B).

12 rabbits were allowed to survive up to 2 months postoperatively. Of these, 6 had been treated with Factor IXai for aortotomy/PTFE graft placement and 6 were treated with heparin. At sacrifice, 100% of the grafts were patent, with no evidence of intimal hyperplasia by hematoxylin and eosin staining, and no evidence of systemic thrombin generation, as measured by Thrombin-antithrombin III complex (TAT).

### B. Canine carotid 2x8 mm PTFE patch repair of Right carotid aortotomy

To date, 14 dogs have undergone this procedure, 7 dogs received Factor IXai (300 microgram/kg) and 7 dogs received heparin (50 IU/kg). The data demonstrate that there is substantially decreased blood loss in dogs treated with Factor IXai vs. those treated with heparin:

| | |
|---|---|
| Factor IXai | 20.8±12.9 gms |
| heparin | 39.1±5.5 gms p<0.05 |

In addition, time to achieve hemostasis was lower in the animals treated with Factor IXai:

| | |
|---|---|
| Factor IXai | 162±34 seconds vs |
| heparin | 228±17 seconds p<0.05 |

See Figures 6A and 6B.

One of the important concerns in such a model is whether or not the agent increased the incidence of graft occlusion or intimal hyperplasia. Similar to the results obtained in rabbits, ultrasound at one month revealed all grafts to be patent; there were no difference observed in dogs treated with Factor IXai or heparin. Also, serial measurement of TAT revealed that there was no evidence of thrombin generation from one week postoperatively, through two months postoperatively.

### II. Modified Cephalin Clotting Time (MCCT)

MCCT has also been characterized.

### A. Limit of detection.

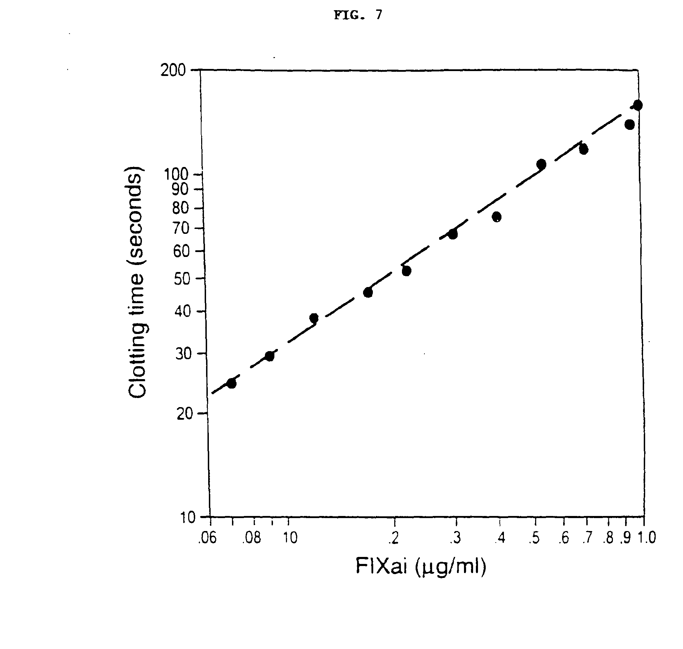

By performing a series of dilutions of added Factor IXai and determining the clotting time as otherwise noted in the original application, it has been determined that the limit of detection of Factor IXai in the assay is 0.4µg/ml. See Figure 7.

### B. Determination of MCCT after single dose of Factor IXai in canine cardiopulmonary bypass.

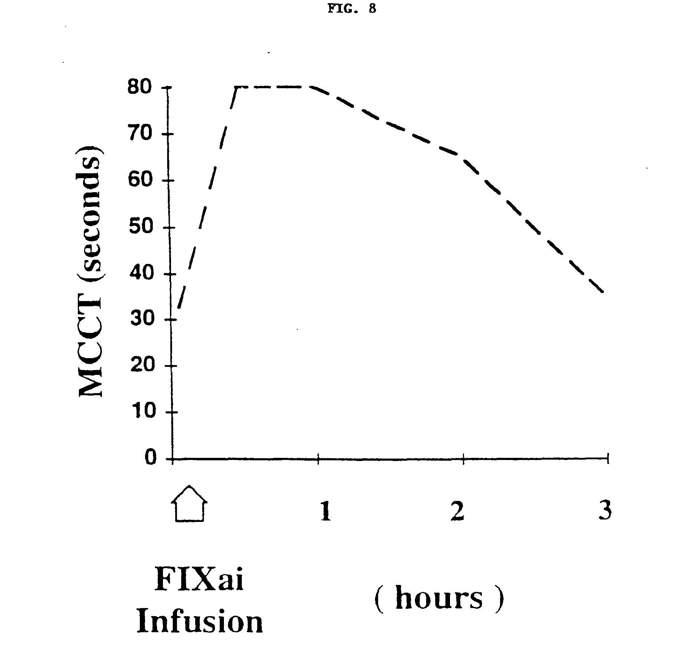

After a single clinically-effective dose of Factor IXai (460µg/kg) in canine CPB, the MCCT rises to 80 secs, and is maintained at the level through at least 1.5 hours of cardiopulmonary bypass (CPB). Post-CPB, MCCT remained elevated through at least 2 hours, and normalized by 3 hours after initiation of CPB. These data suggested that in uncomplicated CPB (at least 1.5 hr), one dose of Factor IXai is likely sufficient. See Figure 8.

### Summary: Active-site Blocked Factor IXa (IX ai): A Novel Selective Anticoagulant for Use in Cardiopulmonary Bypass

The use of heparin in cardiopulmonary bypass (CPB) prevents intravascular/extracorporeal circulation thrombosis but also interferes with protective extravascular hemostasis due to its multiple sites of blockade in the coagulation cascade and its adverse effect on platelet function. In addition, the presence of heparin-associated antibodies can result in paradoxical thrombosis with severe consequences. Furthermore, protamine reversal may be accompanied by allergy, hemodynamic instability, or pathologic thrombosis. An alternative to heparin would have great clinical implications. The prothrombotic stimuli associated with CPB are complex with early activation likely to occur through activation of the contact(intrinsic) pathway. Later in CPB(>2 hrs), activated monocytes on the bypass circuitry express low levels of tissue factor(TF), resulting in activation of Factor IX by Factor VIIa-TF pathway. The role of Factor IXa (IXa) was studied since it is activated upon stimulation of the contact/intrinsic system; as well as in the presence of low amounts of TF, such as that observed early in CPB or later in CPB with low numbers of activated monocytes on the bypass circuitry. It was hypothesized that blockade of IXa would inhibit intravascular/extracorporeal thrombosis while preserving extravascular hemostasis(where high amounts of Factor VIIa-TF will directly activate Factor X and promote clot formation). A baboon model of CPB was established using active site-blocked IXa, (dansyl-glutamyl, glycyl-arginyl chlormethylketone IXai), a competitive inhibitor of the assembly of IXa in the Factor X activation complex, as an anticoagulant. Standard single-stage CPB was performed in 10 baboons; 6 received Ixai(300 to 600 µg/kg)/no reversal and 4 received heparin(300 IU/kg) /protamine(2 mg/kg). CPB was maintained for one hour with cooling to 32°C. An aortotomy was performed and repaired. Baboons were weaned from CPB and blood loss monitored for 3 hours postoperatively. At doses from 400 µg/kg to 460 µg/kg systemic hemodynamic profiles were similar in both groups throughout the procedure, with no evidence of excess pressures in the CPB circuit or gross clot formation in the tubing. Scanning electron microscopy of arterial filters revealed no differences in fibrin deposition in the animals treated with IXai vs. those treated with heparin. At necropsy, there was no evidence of pathologic clot formation or bleeding within the abdominal or thoracic cavities nor fibrin deposition/microemboli in the heart, lung, liver or kidney in animals treated with IXai or heparin. In contrast to heparin, animals treated with IXai (at doses < 600 µg/kg) had hemostatic clot along the cut surface of the sternum and in surgical tissue planes during the entire procedure. Blood samples revealed similar dilutional decreases in hematocrit, platelet count, and fibrinogen levels. Prothrombin time and partial thromboplastin time were only slightly elevated in baboons receiving IXai (13.2 ± 1.2 and 27.1 ± 4.5, respectively). Similarly, activated clotting time (ACT) was unchanged in the IXai group. In order to rapidly and reproducibly measure the effective antithrombotic level of IXai in plasma, a cephalin-IXa-based clotting assay was developed (modified cephalin clotting time; or MCCT). At 1:32 dilution of cephalin, normal test plasma had clotting time of 21 secs. After a single infusion of IXai (400 to 460 µg/kg), MCCT rose to 80 secs and remained at that level for up to 2 hours postoperatively; thereby potentially protecting bypass graft patency into the immediate postoperative period. Taken together, these data suggest that IXai may be a safe and effective alternative anticoagulant for selected patients undergoing CPB, preventing intravscular-extracorporeal circulation thrombosis while preserving extravascular hemostasis.

### References

Ahmad, S., et al. (1989) *J*. *Clin. Invest.* **84**:824-828.
Ahmad, S., et al. (1989) *J. Biol. Chem.* **264:**3244-3251.
Ahmad, S.D., et al. (1992) *J*. *Biol. Chem.* **267(12)**:8571-6.
Astermark, J., et al. (1991) *J*. *Biol. Chem.* **266**:2430-2437.
Astermark, J., et al. (1992) *J*. *Of Biol. Chem.* **267(5)**:3249-56.
Atkinson, J.B., et al.(1992) *J*. *Of Pediatric Surgery* **27(9)**:1197-1198.
Benedict, C., et al. (1991) *J*. *Clin. Invest.* **88**:1760-1765.
Bernabei, A., et al. (1995) *J. Thorac. & Cardiovasc. Surg.* **109(4)**:765-71.
Berntorp, E., et al. (1993) *Thrombosis and Haemostasis* **70(5)**:768-773.
Blondelle et al. (1994).
Brister, S.J., et al. (1994) *Thrombosis & Haemostasis* **71**:468-473.
Chomiak, P.N., et al. (1993) *Circ*. **88**:407-412.
Daly, R.C., et al. (1988) *J*. *Thorac.* & *Cardiovasc. Surg.* **96(1) :**19-29.
DeAnda, A., et al. (1994) *Ann. Thorac. Surg.* **58**:344-350.
Derian, C., et al. (1989) *Biol. Chem.* **264**:6615-6618.
Edmunds, L. H. (1993) *J*. *Card. Surg.* **8**:404-410.
Edmunds, L. H. (1995) *Adv. In Cardiac Surg.* **6**:131-167.
Freedman, S.J., et al. (1995) *J. Biol. Chem.* **270(14):**7980-7987.
Furie, B.C. and Furie, B. (1995) *Thrombosis and Haemostasis* **74(1)**:274-277.
Gitel, S., et al. (1977) *Proc. Natl. Acad. Sci.* **74**:3028-3032.
Gravlee, G.P., et al. (1990) *J. Thorac, Cardiovasc. Surg.* **99**:518-527.
Gurewich, V., et al. (1979) *Thromb. Res.* **14**:931-940.
Heimark, R., and Schwartz, S. (1983) *Biochem. Biophys. Res. Commun.* **111**:723-731.
Iberti, T.J., et al. (1994) *Neurosurgery* **34(3) :**389-395.
Keating, M.T. and Sanguinetti, M.C. (1996) Science 272:681-685.
Kuwabara, K., et al. (1995) *J. Biol. Chem.* **270(14):**8179-8187.
Lollar, P., and Fass, D. (1984) *Arch*. *Biohcem. Biophys.* **233**:673-682.
London, F. and P. Walsh.(1992) *Circulation* **86:** (suppl 1), 1855.
Magovern, G.L. (1995) *Advances in Cardiac Surgery* **6:**169-193.
Miyata, T., et al. (1994) *British J. of Haematology* **88:**156-165.
Muehrcke, D.D., et al. (1995) *J. Thorac. Cardiovasc. Surg.* **110:**843-51.
Perchinisky, M.J., et al.(1995) *Am. J. Surg.* **169**:488-491.
Rimon, S., et al. (1987) *J. Biol. Chem.* **262:**6023-6031.
Ryan, J., et al. (1989) *J. Biol. Chem.* **264:**20283-20287.
Santagostino, E., et al. (1994) *Thrombosis and Haemostasis* **71(6)**:737-740.
Stern, D., et al. (1983) *Proc. Natl. Acad. Sci.* **81**:913-917.
Stern, D., et al. (1987) *Br. J. Hematol.* **66:**227-232.
Stern, D., et al. (1985) *J. Biol. Chem.* **260:**6717-6722.
Thompson, A. (1986) *Blood* **67**:565-572.
Toomey, J*.,* et al. (1991) *J. Biol. Chem.* **266:**19198-19202.
Toomey, J., et al.(1992) *Biochemistry* **31**:1086-1808.
Wacey, A.I., et al. (1994) *Hum. Genet.* **94**:594-608.
Walenga, J.M., et al.(1991) *Ann. Thorac. Surg.* **51**:271-277.
Warrier, I., et al.(1995) *Am. J. of Hematol.* **49**:92-94.
Wickelgren, I. (1996) *Science* **272**:668-670.

## Claims

1. Use of a Factor IXmi or a Factor IXami for the manufacture of a medicament for inhibiting thrombosis in a patient whose blood is subjected to extracorporeal circulation.

2. The use of claim 1, wherein the use is of a Factor IXmi.

3. The use of claim 2, wherein the Factor IXmi is selected from the group consisting of:
(i) Factor IXmi (Ser365-Xxx);
(ii) Factor IXmi (Asp269-Yyy);
(iii) Factor IXmi (His221-Zzz);
(iv) Factor IXmi (Ser365-Xxx, Asp269-Yyy);
(v) Factor IXmi (Ser365-Xxx, His221-Zzz);
(vi) Factor IXmi (Asp269-Yyy, His221-Zzz);
(vii) Factor IXmi (Ser365-Xxx. Asp269-Yyy, His221-Zzz): and
(viii) Factor IXmi (Ser365-Ala)

4. The use of claim 1, wherein the use is of a Factor IXami,

5. The use of claim4, wherein the Factor IXami is selected from the group consisting of
(i) Factor IXami (Ser365-Xxx);
(ii) Factor IXami (Asp265-Yyy);
(iii) Factor IXami (His221-Zzz);
(iv) Factor IXami (Ser365-Xxx. Asp269-Yyy);
(v) Factor IXami (Ser365-Xxx, His221-Zzz);
(vi) Factor IXami (AspZ69-Yyy, His221-Zzz);
(vii) Factor IXami (Ser365-Xxx, Asp269-Yyy, His221-Zzz); and
(viii) Factor IXami (Ser365-Ala).

6. The use of claim 1. wherein the Factor IXmi or Factor IXami is to be administered at a concentration of from about 0.01 µg/ml plasma to about 250 µg/ml plasma.

7. The use of claim 1, wherein the Factor IXmi or Factor IXami is to be administered at a concentration of from about 0.05 µg/ml plasma to about 25 µg/ml plasma.

8. The use of claim 1, wherein the Factor IXmi or Factor IXami is to be administered at a concentration of from about 0.1 µg/ml plasma to about 5 µg/ml plasma.

9. The use of claim 1, wherein the patient is subjected to extracorporeal blood circulation during transplant surgery, abdominal surgery, vascular surgery or a cardiopulmonary bypass surgery.

10. The use of claim 1, wherein the patient is a human being.

## Patentansprüche

1. Verwendung eines Faktors IXmi oder eines Faktors IXami zur Herstellung eines Medikaments zum Inhibieren von Thrombose in einem Patienten, dessen Blut extrakorporaler Zirkulation unterzogen wird.

2. Verwendung nach Anspruch 1, wobei die Verwendung die eines Faktors IXmi ist.

3. Verwendung nach Anspruch 2, wobei der Faktor IXmi ausgewählt ist aus der Gruppe bestehend aus:
(i) Faktor IXmi (Ser365-Xxx);
(ii) Faktor IXmi (Asp269-Yyy);
(iii) Faktor IXmi (His221-Zzz);
(iv) Faktor IXmi (Ser 365-Xxx, Asp269-Yyy);
(v) Faktor IXmi (Ser365-Xxx, His221-Zzz);
(vi) Faktor IXmi (Asp269-Yyy, His221-Zzz);
(vii) Faktor IXmi (Ser365-Xxx, Asp269-Yyy, His221-Zzz); und
(viii) Faktor IXmi (Ser365-Ala)

4. Verwendung nach Anspruch 1, wobei die Verwendung die eines Faktors IXami ist.

5. Verwendung nach Anspruch 4, wobei der Faktor IXami ausgewählt ist aus der Gruppe bestehend aus:
(i) Faktor IXami (Ser365-Xxx);
(ii) Faktor IXami (Asp265-Yyy);
(iii) Faktor IXami (His221-Zzz);
(iv) Faktor IXami (Ser365-Xxx, Asp269-Yyy);
(v) Faktor IXami (Ser365-Xxx, His 221-Zzz);
(vi) Faktor IXami (Asp269-Yyy, His 221-Zzz);
(vii) Faktor IXami (Ser365-Xxx, Asp269-Yyy, His221-Zzz); und
(viii) Faktor IXami (Ser365-Ala).

6. Verwendung nach Anspruch 1, wobei der Faktor IXmi oder Faktor IXami in einer Konzentration von ungefähr 0.01 µg/ml Plasma bis ungefähr 250 µg/ml Plasma zu verabreichen ist.

7. Verwendung nach Anspruch 1, wobei der Faktor IXmi oder Faktor IXami in einer Konzentration von ungefähr 0.05 µg/ml Plasma bis ungefähr 25 µg/ml Plasma zu verabreichen ist.

8. Verwendung nach Anspruch 1, wobei der Faktor IXmi oder Faktor IXami in einer Konzentration von ungefähr 0.1 µg/ml Plasma bis ungefähr 5 µg/ml Plasma zu verabreichen ist.

9. Verwendung nach Anspruch 1, wobei der Patient einem extrakorporalen Blutkreislauf während Transplantationschirurgie, Abdominalchirurgie, Gefäßchirurgie oder einer kardiopulmonalen Bypasschirurgie unterzogen wird.

10. Verwendung nach Anspruch 1, wobei der Patient ein Mensch ist.

## Revendications

1. Utilisation d'un facteur IXmi ou d'un facteur IXami pour la fabrication d'un médicament destiné à inhiber la thrombose chez un patient dont le sang est soumis à une circulation extracorporelle.

2. Utilisation selon la revendication 1, dans laquelle l'utilisation est celle d'un facteur IXmi.

3. Utilisation selon la revendication 2, dans laquelle le facteur IXmi est choisi dans le groupe constitué par :
(i) le facteur IXmi (Ser365-Xxx) ;
(ii) le facteur IXmi (Asp269-Yyy) ;
(iii) le facteur IXmi (Hia221-Zzz) ;
(iv) le facteur IXmi (Ser365-Xxx, Asp269-Yyy) ;
(v) le facteur IXmi (Ser365-Xxx, His221-Zzz) ;
(vi) le facteur IXmi (Asp269-Yyy, His221-Zzz) ;
(vii) le facteur IXmi (Ser365-Xxx, Asp269-Yyy, His221-Zzz) ; et
(viii) le facteur IXmi(Ser365-Ala).

4. Utilisation selon la revendication 1, dans laquelle l'utilisation est celle d'un facteur IXami.

5. Utilisation selon la revendication 4, dans laquelle le facteur IXami est choisi dans le groupe constitué par :
(i) le facteur IXami (Ser365-Xxx) ;
(ii) le facteur IXami (Asp269-Yyy) ;
(iii) le facteur IXami (His221-Zzz) ;
(iv) le facteur IXami (Ser365-Xxx, Asp269-Yyy) ;
(v) le facteur IXami (Ser365-Xxx, His221-Zzz) ;
(vi) le facteur IXami (Asp269-Yyy, His221-Zzz) ;
(vii) le facteur IXami (Ser365-Xxx, Asp269-Yyy, His221-Zzz) ; et
(viii) le facteur IXami(Ser365-Ala).

6. Utilisation selon la revendication 1, dans laquelle le facteur IXmi ou le facteur IXami doit être administré à une concentration d'environ 0,01 µg/ml de plasma à environ 250 µg/ml de plasma.

7. Utilisation selon la revendication 1, dans laquelle le facteur IXmi ou le facteur IXami doit être administré à une concentration d'environ 0,05 µg/ml de plasma à environ 25 µg/ml de plasma.

8. Utilisation selon la revendication 1, dans laquelle le facteur IXmi ou le facteur IXami doit être administré à une concentration d'environ 0,1 µg/ml de plasma à environ 5 µg/ml de plasma.

9. Utilisation selon la revendication 1, dans laquelle le patient est soumis à une circulation sanguine extracorporelle pendant une chirurgie de transplantation, une chirurgie abdominale, une chirurgie vasculaire ou une chirurgie de pontage cardiopulmonaire.

10. Utilisation selon la revendication 1, dans laquelle le patient est un être humain.
